# EUROPEAN PATENT SPECIFICATION

(11) **EP 2 853 384 B1**
(45) Date of publication and mention of the grant of the patent: **23.11.2016**
(21) Application number: 13186335.9
(22) Date of filing: 27.09.2013
(51) Int. Cl.: B29C 70/86, A61L 27/34, A61F 2/30

(54) **A method for coating and a coated surface**
Verfahren zur Beschichtung und eine beschichtete Oberfläche
Procédé de revêtement et surface revêtue

(43) Date of publication of application: 01.04.2015
(73) Proprietor: Skulle Implants OY, 20520 Turku (FI)
(72) Inventor: Karhi, Olli, 90230 Oulu (FI); Vallittu, Pekka, 21620 Kuusisto (FI); Nuutinen, Juha-Pekka, 33210 Tampere (FI)
(74) Representative: Suominen, Kaisa Liisa

(56) References cited:
- EP-A1- 1 611 906
- WO-A1-2005/018698

## Description

### FIELD OF THE INVENTION

The present invention relates to a method of coating a surface as well as to a coated surface and a coated implant.

### BACKGROUND OF THE INVENTION

Metallic implants, such as titanium, titanium-aluminium, vanadium, tantalium, cobalt-chromium and cobalt-chromium-molybdenium implants are widely used especially in orthopaedics. There are also implants made of ceramics such as zirconia and implants made of plastics (often reinforced with fibres) such as polyetherethercetone. A problem encountered especially with load bearing implants such as hip prostheses and knee prostheses is the attachment of the metallic implant to the surrounding bone. Typically the implant is attached to the bone by using bone cement, press-fit without cement or with screw fixation. There exists however still a need to provide more reliable attachment means that provide for long term stability. Roughening the surface of the implant or changing the surface topography of the implant surface have also been proposed for this purpose.

On the other hand, a lot of development has occurred with bioactive materials, namely bioactive ceramics and glass and sol-gel processed silica. These materials can be used to achieve attachment of e.g. bone to a biomaterial surface after the material has been put in contact with tissue. An additional advantage of bioactive glass is its antimicrobial effect on the microbes existing for instance in infected sinuses of a bone or in case of osteomyelitis.

### OBJECTS AND SUMMARY OF THE INVENTION

An object of the present invention is to provide a biologically and mechanically compatible coating for implant materials that does not have the above-listed drawbacks, or at least those disadvantages are minimised. Specifically, an object of the present invention is to provide a method of coating a surface that enhances the adhesion and attachment of the surface to bone. Another object of the invention is to provide a coated surface and a coated implant for use in bone surgery and dental surgery.

WO2005/018698 discloses a method, comprising the steps of
- forming and/or arranging a composite structure, the composite structure comprising
- a first fibre fabric impregnated with a first resin,
- at least one fibre bundle impregnated with a second resin, the fibre bundles being part of additional fabric, the fibre bundles being arranged in a fabric thereby forming in-between spaces, and in contact with the first fibre fabric,
- particles of bioactive material arranged into said in-between spaces of the fibre bundle and arranged in contact with the first fibre fabric,
- a second fibre fabric impregnated with a third resin and arranged in contact with the fibre bundles and at least part of the particles of bioactive material - polymerising the resins of the composite structure.

A typical method according to the present invention for coating a surface comprises the steps of
- abrading the surface to be coated with particles to form an abraded surface,
- forming and/or arranging a composite structure on the abraded surface, the composite structure comprising
   - a first fibre fabric impregnated with a first resin,
   - at least one fibre bundle impregnated with a second resin, the fibre bundle being arranged according to a pattern forming in-between spaces, and in contact with the first fibre fabric,
   - particles of bioactive material arranged into said in-between spaces of the fibre bundle and arranged in contact with the first fibre fabric,
   - a second fibre fabric impregnated with a third resin and arranged in contact with the fibre bundles and at least part of the particles of bioactive material, wherein the mesh size of the second fibre fabric is smaller than the average diameter of the particles of bioactive material,
- polymerising the resins of the composite structure.

### BRIEF DESCRIPTION OF THE DRAWING

- Figure 1: schematically shows a finished coating according to a first embodiment.
- Figures 2a and 2b: schematically show a total hip prostheses coated according to a second embodiment.
- Figure 3: schematically shows a dental implant coated according to a third embodiment.
- Figures 4a and 4b: schematically show an acetabular cup system coated according to a fourth embodiment.
- Figure 5: schematically shows a knee implant coated according to a fifth embodiment.
- Figure 6: schematically shows a fracture fixation plate fixed by screws to a clavicle bone.

### DETAILED DESCRIPTION OF THE INVENTION

A typical method of coating a surface according to the present invention comprises the steps of
- abrading the surface to be coated with particles to form an abraded surface,
- forming and/or arranging a composite structure on the abraded surface, the composite structure comprising
   - a first fibre fabric impregnated with a first resin,
   - at least one fibre bundle impregnated with a second resin, the fibre bundle being arranged according to a pattern forming in-between spaces, and in contact with the first fibre fabric,
   - particles of bioactive material arranged into said in-between spaces of the fibre bundle and arranged in contact with the first fibre fabric,
   - a second fibre fabric impregnated with a third resin and arranged in contact with the fibre bundles and at least part of the particles of bioactive material, wherein the mesh size of the second fibre fabric is smaller than the average diameter of the particles of bioactive material,
- polymerising the resins of the composite structure.

The method thus provides a coating to a surface such as an implant surface, where firstly the surface of the implant is treated to enhance the attachment of the following layers to the surface of the implant, and then the following layers are constructed in such a manner as to provide both good adhesion and attachment to bone as well as good load bearing capabilities to the combination implant and bone. The implant can of course also adhere well to cartilage. The coating according to the present invention can naturally be used for other surfaces than implant surfaces, but it is believed to be especially suitable for these purposes.

The surface to be coated is preferably a macroscopically appropriate surface for mechanically interlocking to the finished coating. Macroscopical refers here to sizes that are approximately 1 mm or larger. The surface to be coated thus preferably has protrusions or the like that have for example a height of about 1 mm. Such surface enables further mechanical retention of the coating to the surface, in addition to the abrasion.

The coating has thus three main layers. A first layer is manufactured by abrading the surface of the implant and attaching the first fibre fabric. The abraded surface gives good adhesion to the first fibre fabric which is attached to the abraded surface via the first resin. The second layer comprises the fibre bundle or bundles and the bioactive particles. The resin impregnated fibre bundle is applied according to a pattern, meaning that typically the whole surface is not covered with the fibre bundle. The fibre bundle forms what can also be called an interconnective element, meaning that it is in contact with both the first fibre fabric and the second fibre fabric. The rest of the surface of the implant is typically mostly covered with particles of bioactive material. This second or core layer is typically the layer that gives the strength to the coating via the fibre bundles. This second layer is also the layer into which bone or cartilage is grown, due to the bioactive particles, thus its thickness is chosen according to the use of the implant; for example, in a dental implant the thickness does not need to be as high as in a knee implant, for example, as the forces to which a knee implant is subjected are far greater than the forces to which a dental implant is subjected.

A third layer of the coating is then the second fibre fabric impregnated with the third resin that is applied to cover the fibre bundle and bioactive particles and the function of this third layer is to provide a surface that allows the implant to be easily handled and to keep the fibre bundle and the particles in place. A further function of the third layer is to allow body fluids to infiltrate the second layer, in order to allow for bone ingrowth. Therefore, it is the third layer typically comprises pores or openings, the size of which is in this application called the mesh size. The second fibre fabric may thus be for example a woven fabric having a grid-like structure or a non-woven fibre fabric that has openings in the form of pores. The mesh size is typically the largest dimension of the opening.

The present invention thus provides a coating for an implant, for example, that allows for a continuous gradient of elastic modulus from the elastic modulus of the implant material towards that of bone, once bone has grown into the composite material. In a preferred embodiment, the surface has mechanical retention surface ensuring the mechanical interlocking of the coating and the surface is further treated by abrasion and by a chemical treatment to ensure appropriate attachment of the coating to the surface.

In this specification, by curing it is meant polymerisation and/or crosslinking. It is also to be understood that for example in the wording "third resin impregnated fibre bundle", the term "third" refers to the resin, not to the fibre bundle, and that the fibre bundle is impregnated with said third resin. The various embodiments and variations explained below will apply *mutatis mutandis* to the method, the coating and the implant.

According to different embodiments, the composite structure can be formed directly onto the surface to be coated, step by step, or the composite structure can be formed separately and then applied to the surface to be coated. Furthermore, the surface to be coated may comprise macromechanically retentive forms, such as extensions protruding from the surface. These extensions may have for example a diameter of from 50 µm to 3 mm and a length of from 50 µm to 5 mm, preferably their sizes are about 1 mm or more. When such extensions are provided on the surface to be coated, the fibre bundle or bundles are preferably arranged such that the thickness of the coating layer remains essentially constant. The extensions may also have such a shape and size that when porous fibre fabrics are used, the extensions can pass through the pores of the fibre fabric. These extensions further increase the mechanical interlocking of the composite structure to the surface to be coated. Furthermore, the fibre bundles can be arranged in such a manner that the extensions penetrate at least partly (or fully) into them, which yet further increases the mechanical interlocking.

When a resin or a fibre fabric or an impregnated fibre fabric is applied onto the surface, it essentially covers the whole surface that comes into contact with bone or cartilage when in use. This means that for example in the case of a dental implant, the coating essentially covers the part of the implant that is inserted into the bone of the patient, but not the part that makes up the missing tooth.

The fibres may be any suitable fibres known *per se,* for example selected from the group consisting of glass fibres, silica fibres, carbon/graphite fibres, ceramic fibres, aramid fibres, zylon fibres, polyethylene fibres, polytetrafluoroethylene fibres, such as Teflon® fibres, poly(p-phenylene-2,6-benzobisoxazole) fibres, poly(2,6-diimidazo(4,5-b4',5'-e)pyridinylene-1,4(2,5-dihydro)phenylene fibres, polyolefin fibres, fibres prepared from copolymers of olefins, polyester fibres, polyamide fibres and mixtures thereof. Poly(p-phenylene-2,6-benzobisoxazole) fibres and poly(2,6-diimidazo(4,5-b4',5'-e)pyridinylene-1,4(2,5-dihydro)phenylene fibres belong to a group called rigid-rod polymer fibres. It is obvious to a person skilled in the art that any other known fibres may be used in the present invention, provided that it is possible to obtain a suitable adhesion between said fibres and the cured resin, in order to achieve the desired mechanical properties.

According to an embodiment, the fibres used in the fabrics and/or the rowing are glass fibres. The glass fibres may be made of a glass composition of S-glass, E-glass or bioactive glass, non-biodegradable fibres being preferred. The fibres in the different fabrics and/or the fibre bundle can be same or different, and several different types of fibres can be used in one fabric and/or fibre bundle. The fabrics may be woven fabrics or nonwoven fabrics.

The fibre bundle may be in the form of a bundle of unidirectional fibres (or filaments) or a fibre rowing. In some embodiments, even a single fibre could be used, although it is believed that better results are obtained when using several fibres, as their interspaces are then also covered with the resin and the polymerisation attaches the fibres to each other, too. The total surface of a fibre bundle is also higher than that of a single fibre, even for an identical total diameter. The fibre bundle may cover from 1 to 95 % of the total surface of the metallic material. The fibre bundle may thus cover from 1, 3, 5, 10, 15, 20, 25, 30, 35, 40, 45 or 50 % up to 5, 10, 15, 20, 25, 30, 35, 40, 45, 50, 55, 60, 65, 70, 75, 80, 85, 90 or 95 % of the total surface. Most typically the fibre bundle covers about 3-10 % of the total surface.

The fibre bundles are preferably arranged on the surface such that they are spaced apart from each other. The distance from one fibre bundle to another depends on the embodiment, but can be for example from 0.1 to 25 mm. The distance can thus be from 0.1, 0.5, 1, 1.5, 2, 5, 8, 10, 12, 15 or 20 mm up to 0.5, 1, 1.5, 2, 5, 8, 10, 12, 15, 20 or 25 mm.

The fibre bundles are arranged onto the surface according to a pattern that forms in-between spaces to the surface. This means that even when only one fibre bundle is used, there are still spaces between the fibre bundles, i.e. for example the fibre bundle is wound around a surface in the form of a spiral. In other embodiments, the fibre bundles form a grid that can be made of for example two, three or four fibre bundles.

In applications, where the implant is subjected to strong forces, the fibre bundle is most typically applied such that it will be at an angle of approximately +/- 45° with respect to the main direction of load of the implant once it is in place in a patient. In other embodiments, it may be applied according to another pattern, for example in the case of an acetabular cup, it may be arranged circularly following the contour of the cup.

According to an embodiment, the diameter of the fibre bundle is 0.1-10 mm. The diameter of the fibre bundle may be for example from 0.1, 0.2, 0.3, 0.5, 0.8, 1, 1.5, 1.8, 2, 2.5, 3, 3.5, 4, 4.5, 5, 5.5, 6, 6.5, 7, 7.5 or 8 mm up to 0.2, 0.3, 0.5, 0.8, 1, 1.5, 1.8, 2, 2.5, 3, 3.5, 4, 4.5, 5, 5.5, 6, 6.5, 7, 7.5, 8, 8.5, 9, 9.5 or 10 mm. The diameter of the fibre bundle typically defines the thickness of the second layer.

According to an embodiment, the bioactive material is selected from the group consisting of bioactive glass, hydroxyapatite, tricalciumphosphate and mixtures thereof. When bioactive glass is used, it may be any bioactive glass known as such, for example the glass S53P4 containing 23-wt-% of Na₂O, 20 wt-% of CaO, 4 wt-% of P₂O₅ and 53 wt-% of SiO₂. Furthermore, other ceramics or silicate materials such as combeite may be used.

The average particle size of the bioactive material may be for example 10-1000 µm. The average particle size of the bioactive material may thus be from 10, 20, 50, 70, 90, 100, 150, 200, 300, 350, 450, 500, 550, 600, 700, 750, or 800 up to 20, 30, 50, 70, 80, 90, 100, 120, 150, 200, 300, 350, 450, 500, 550, 600, 700, 750, 800, 850, 900, 950 or 1000 µm.

The bioactive particles may cover from 5 to 99 % of the total surface of the metallic material. The particles may thus cover from 5, 10, 15, 20, 25, 30, 35, 40, 45 or 50 % up to 10, 15, 20, 25, 30, 35, 40, 45, 50, 55, 60, 65, 70, 75, 80, 85, 90 or 99 % of the total surface. Most typically the particles cover about 80-97 % of the total surface, while bearing in mind that not all of the surface needs to be covered by either the fibre bundle or particles, but that there can and preferably are also voids between the particles. The second fibre fabric is also not necessary in contact with all the bioactive particles, but is preferably in contact with at least a part of them, even more preferably a majority of them.

According to an embodiment, the step of arranging the composite structure on the abraded surface comprises the steps of
- applying the first resin to the abraded surface to form a first resin coated surface,
- applying the first fibre fabric onto the first resin coated surface,
- polymerising the first resin in atmospheric conditions to form an oxygen inhibited surface,
- applying the at least one fibre bundle impregnated with the second resin according to the pattern to the oxygen inhibited surface,
- applying particles of bioactive material to the oxygen inhibited surface into said in-between spaces of the fibre bundle,
- polymerising the second resin to form a semi-coated surface,
- applying the second fibre fabric impregnated with the third resin to the semi-coated surface, and
- polymerising the third resin of the second fibre fabric to form the coated surface.

According to another embodiment, the composite structure is formed separately and arranged on the abraded surface. In this case, the composite structure can be formed in any suitable way, for example in an opposite order to that explained above: firstly providing the second fibre fabric, arranging the fibre bundle(s) and the bioactive particles to the second fibre fabric and applying the first fibre fabric as a last step. Moreover, the resins can be polymerised each individually, before applying a further layer of the composite or two or all at the same time. For example, it could be envisaged that the composite is manufactured by providing the second fibre fabric and third resin, polymerising the third resin, then arranging the fibre bundle(s) and the bioactive particles to the second fibre fabric and polymerising the second resin, whereafter the first fibre fabric is applied and the first resin polymerised once the composite structure has been arranged on the surface to be coated.

According to an embodiment, the method further comprises a step of post-curing the coated surface. This post-curing (also called post-polymerisation) is carried out in conditions suitable for the resins used, typically at elevated temperatures such as from 50 to 120 °C, depending on the glass transition temperature of the polymer. The post-curing may be carried out in total or partial vacuum or under atmospheric pressure conditions.

According to another embodiment, the first fibre fabric is impregnated with the first resin prior to applying it to the abraded surface. This means that it is possible to firstly impregnate the first fibre fabric and then to apply it to the abraded surface. Indeed, any of the other steps including applying a resin and a fibre product may also be carried out separately (applying firstly the fibre product and then the resin) or concurrently (firstly impregnating the fibre product with the resin and then applying the impregnated fibre product), although in most of the steps of the method, it is practically easiest to firstly impregnate the fibre product with the resin.

According to a preferred embodiment, the abrasion is carried out by air-particle abrasion. These particles may be any particles suitable *per se,* i.e. having the required hardness.

When the abraded surface is to be silanated, the particles used are preferably particles containing silica. Some examples of suitable particles are silica coated alumina particles, such as used in the tribochemical silica coating-method (Rocatec and Coejet) by 3M-ESPE, Seefeld, Germany. When the abrasion is carried out with particles containing silica, the abraded surface will contain silica, whereby the solution containing a silane coupling agent, once it is polymerised, leads to a silanized surface

According to yet another embodiment, the step of abrading the surface to be coated is thus carried out with particles containing silica and the method further comprises, after abrading the surface to be coated and prior the application of the composite structure, the steps of
- applying a solution containing a silane coupling agent to the abraded surface to form a solution coated surface, and
- polymerizing the solution to form a silanized surface.

According to another embodiment, the solution containing a silane coupling agent is allowed to hydrolyse before applying it to the abraded surface. The hydrolysation reaction may be allowed to carry out for example in room temperature for one hour, the person skilled in the art being readily able to know the time and conditions required. With some solutions, the pH of the solution also needs to be adjusted.

According to yet another embodiment, the step of polymerising the silane solution is carried out in an elevated temperature suitable for polymerisation of the solution. For example, this step may be carried out at a temperature of 50-150 °C for 0.5-2 hours. A person skilled in the art is readily able to determine the conditions necessary for the solution to obtain a suitable degree of polymerisation, such as for example for 95 % of the monomers to have reacted.

The first, second and third resins may be heat-polymerisable or photopolymerisable resins. They may be different or identical or two of them may be identical and one different. These resins may be selected from the group consisting of substituted and unsubstituted dimethacrylates and methacrylates. Some especially advantageous resins are methyl acrylate, methyl methacrylate, methacrylate functionalized dendrimers, glycidyl dimethacrylate (bis-GMA), triethylene glycol dimethacrylate (TEGDMA) and urethane dimethacrylate (UDMA). The materials may be used as blends and they may form interpenetrating polymer networks (IPNs). They may also be functionalised with bioactive molecules that allow for a drug-like contact effect. Combinations of monomers and polymers are also suitable to be used, including modifications of resin systems by antimicrobial side group containing iodine which offers additional benefit in increasing radio opacity of the resin system.

The resins may further comprise monomers selected from the group consisting of methyl acrylate, ethyl acrylate, propyl acrylate, isopropyl acrylate, n-hexyl acrylate, styryl acrylate, allyl acrylate, methyl methacrylate, ethyl methacrylate, propyl methacrylate, isopropyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diurethane dimethacrylate, acetoacetoxy ethyl methacrylate (AAEM), methacrylate functionalized dendrimers, other methacrylated hyperbranched oligomers, hydroxymethyl methacrylate, hydroxymethyl acrylate, hydroxyethyl methacrylate, hydroxyethyl acrylate, hydroxypropyl methacrylate, hydroxypropyl acrylate, tetrahydrofurfuryl methacrylate, tetrahydrofurfuryl acrylate, glycidyl methacrylate, glycidyl acrylate, triethylene glycol diacrylate, tetraethylene glycol dimethacrylate, tetraethylene glycol diacrylate, trimethylolethane trimethacrylate, trimethylolpropane trimethacrylate, pentaerythritol trimethacrylate, trimethylolethane triacrylate, trimethylolpropane triacrylate, pentaerythritol triacrylate, pentaerythritol tetramethacrylate, pentaerythritol tetra-acrylate, ethylene dimethacrylate, ethylene diacrylate, ethylene glycol dimethacrylate, diethylene glycol dimethacrylate, triethylene glycol dimethacrylate (TEGDMA), ethylene glycol diacrylate, diethyleneglycol diacrylate, butylene glycol dimethacrylate, butylene glycol diacrylate, neopentyl glycol dimethacrylate, neopentyl glycol diacrylate, 1,3-butanediol dimethacrylate, 1,3-butanediol diacrylate, 1,4-butanediol dimethacrylate, 1,4-butanediol diacrylate, 1,6-hexanediol dimethacrylate, 1,6-hexanediol diacrylate, di-2-methacryloxyethyl-hexametylene dicarbamate, di-2-methacryloxyethyl-trimethylhexametylene dicarbamate, di-2-methacryloxyethyl-dimethylbenzene dicarbamate, di-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-trimethylhexamethylene dicarbamate, di-1-chloromethyl-2-methacryloxyethyl-dirnethylbenzene dicarbamate, di-1-chloromethyl-2-methacryloxyethyl-dimethylcyclohexane dicarbamate, methylene-bis-1-chloromethyl-2-methacryloxyethyl-4-cyclohexyl carbamate, 2,2-bis(4-(2-hydroxy-3-methacryloxy)phenyl)propane (BisGMA), 2,2'-bis(4-methacryloxyphenyl)propane, 2,2'-bis(4-acryloxyphenyl)propane, 2,2'-bis[4(2-hydroxy-3-acryloxyphenyl)propane, 2,2'-bis(4-methacryloxyethoxyphenyl)propane, 2,2'-bis(4-acryloxyethoxyphenyl)-propane, 2,2'-bis(4-methacryloxypropoxyphenyl)propane, 2,2'-bis(4-acryloxy-propoxyphenyl)propane, 2,2'-bis(4-methacryloxydiethoxyphenyl)-propane, 2,2'-bis(4-acryloxydiethoxyphenyl)propane, 2,2'-bis[3(4-phenoxy)-2-hydroxypropane-1-methacrylate]propane, 2,2'-bis[3(4-phenoxy)-2-hydroxypropane-1-acrylate]propane and mixtures thereof.

The resins may also contain crosslinkable monomers or polymers such as ε-caprolactone, polycaprolactone, polylactides, polyhydroxyproline, and other biopolymers as well as polyamides, polyurethane, polyethylene, polypropylene, other polyolefins, polyvinyl chloride, polyester, polyether, polyethyleneglycol, polysaccharide, polyacrylonitrile, poly(methyl methacrylate), phenol-formaldehyde, melamine-formaldehyde, and urea-formaldehyde.

Dendrimers having 5 to 35 functional groups (or more) such as methacrylate or acrylate groups, may also be used. Multifunctionality forms highly cross-linked matrix and decreases the creep of the polymer in the long-term use. The functionality of the dendrimers can be changed to be suitable for attaching drug molecules to the dendrimer based polymer for allowing local slow drug release from the dendrimer based implant. Examples of suitable dendrimers are given for example in US 5,834,118. Dendrimers may particularly be starburst or hyperbranched methacrylated polyesters.

According to one embodiment, the resins can comprise monomer systems of mono-, bi-, or multifunctional acrylates, epoxies, dendrimers, hyperbranched reactive polymers, their combinations, or the like. The resins may, for example, be selected from the group consisting of mono-, di- and multifunctional acrylates, mono-, di- and multifunctional methacrylates, epoxies, starburst methacrylated polyesters, hyperbranched methacrylated polyesters and mixtures thereof. Optionally, polymers of polymethyl methacrylate, polyvinyl chloride, polyetherketone, polylactides, epsiloncaprolactone or their combinations, or the like may be used.

According to an embodiment, the resins are selected from the group consisting of triethylene glycol dimethacrylate, 2,2-bis(4-(2-hydroxy-3-methacryloxy)phenyl)propane, polymethyl methacrylate, methyl methacrylate, hydroxyethyl methacrylate, urethan dimethacrylate, starburst methacrylated polyesters, hyperbranched methacrylated polyesters, polyvinyl chloride, polyetherketone, polylactides, ε-caprolactone, poly-OH-proline and mixtures thereof.

The bioactive material may also, in addition to the particles, contain material in the form of a fluid having a viscosity such that the layers of mesh are impermeable to the fluid. The fluid can be a highly viscous fluid or a colloid in fluid form. By colloid, it is meant a substance microscopically dispersed evenly throughout another substance.

The coating may further comprise additional particulate filler material, such as metal oxides, ceramics, polymers and mixtures thereof. Metal oxides may for example be used as radio or X-ray opaque materials or as colouring materials.

The coating may also comprise therapeutically active agents or cells such as stem cells, peptides, proteins such as growth factors and/or signalling molecules. Several kinds of cells including hematopoietic bone marrow cells, fibroblasts, osteoblasts, regenerative cells, stem cells, like embryonic stem cells, mesenchymal stem cells or adipose stem cells can be seeded to the coating. If embryonic stem cells are used, they are not of a human origin. Stem cells seeded to the coating can be cultured in bioreactors ex vivo, in other parts of the body before inserting the formed tissue into its final place, or directly at the place where regenerative and reconstructive treatment is needed. The coating may contain also additives enhancing its processability, such as polymerisation initiators.

According to one embodiment, the method comprises the steps of
a) abrading the surface to be coated with particles containing silica to form an abraded surface,
b) applying a solution containing a silane coupling agent to the abraded surface to form a solution coated surface,
c) polymerizing the solution to form a silanized surface,
d) applying a first resin to the silanized surface to form a first resin coated surface,
e) applying a first fibre fabric onto the first resin coated surface,
f) polymerising the first resin in atmospheric conditions to form an oxygen inhibited surface,
g) applying a second resin impregnated fibre bundle according to a pattern to the oxygen inhibited surface,
h) applying particles of bioactive material to the oxygen inhibited surface between the fibre bundles,
i) polymerising the second resin to form a semi-coated surface,
j) applying a third resin impregnated second fibre fabric to the semi-coated surface, wherein the mesh size of the second fibre fabric is smaller than the average diameter of the particles of bioactive material, and
k) polymerising the third resin of the second fibre fabric to form a coated surface.

The method may also comprise a further step I) sterilizing the finished, coated device. Sterilisation may be carried out for example by gamma-radiation, hot air, ethylene oxide gas or hydrogen peroxide plasma.

The invention further relates to a coating of a surface obtainable by the present method. As has been explained above, coating an implant according to the present method yields implants with enhanced attachment properties to bone and cartilage, thus increasing the time of use of the implants. Indeed, when an implant needs to be replaced, this is usually not due to the fact that the implant itself is worn out, but instead to the fact that the implant loosens. Therefore, with the present invention, prosthesis patients will require less surgery, leading to a better quality of life for these patients and decreased health costs for the society.

The invention still relates to an implant obtainable by at least partially coating its surface according to the present method. The implant may be made of metal, such as titanium, cobalt-chromium or tantalium, ceramics such as zirconia and plastics (often reinforced with fibres) such as polyetherethercetone. It may be selected from the group consisting of hip prostheses, acetabular cups, knee prostheses, intramedullary nails, fracture fixation plates and dental implants. Any other types of implants used in humans or animals in surgery or dentistry may be coated according to the present method. A particular advantage is achieved when implants that will be load-bearing once installed, are coated, as the present coating method provides for a good adhesion and attachment of the implant to surrounding bone or cartilage. Typically, the part of the implant that comes into contact with bone or cartilage is coated according to the present invention.

Some embodiments of the invention are explained in more detail in the enclosed drawing, which is not to be construed as limiting the claims. The reference signs are also not to be construed as limiting the claims. It is furthermore to be noted that when dimensions are given in the description, they are not necessarily shown in the corresponding Figure, for sake of clarity.

### DETAILED DESCRIPTION OF THE DRAWING

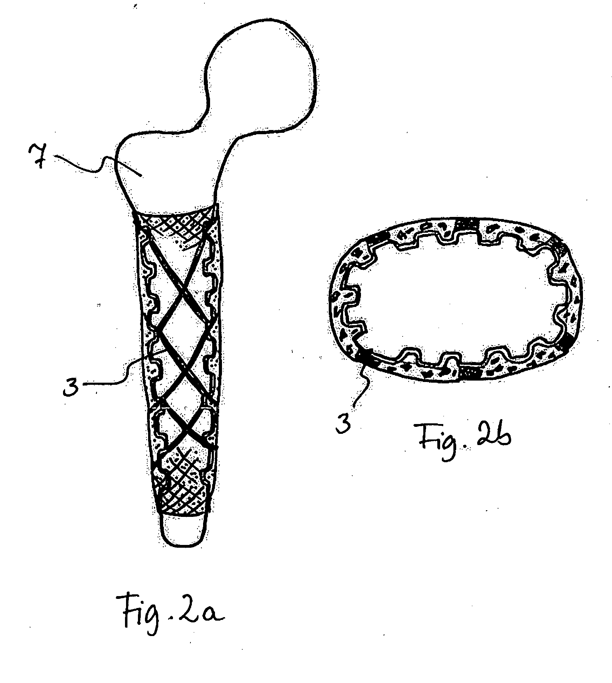

In the following, the same reference signs are used of the same or similar components in different embodiments and/or Figures. In Figures 2 to 5, the embodiment is shown as a partial cross-section.

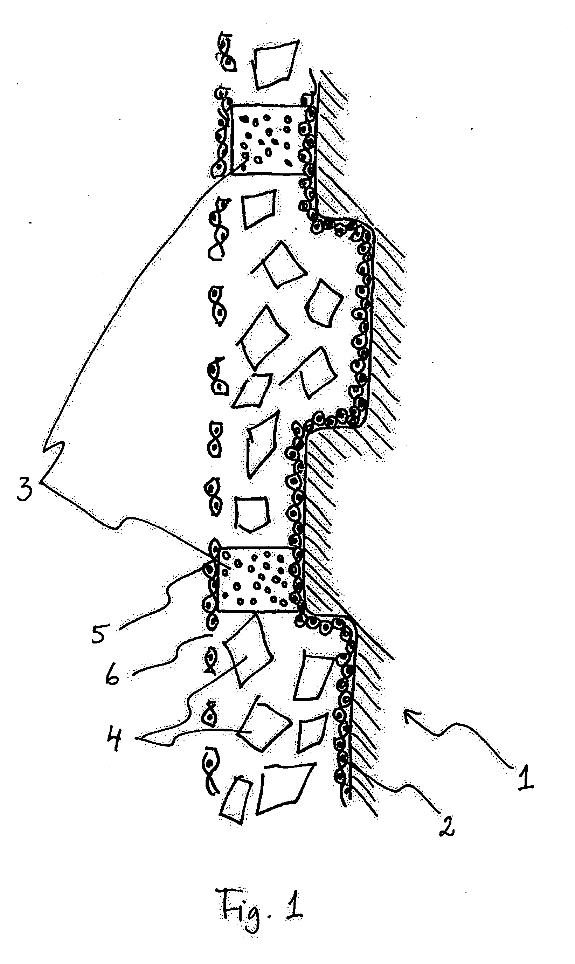

Figure 1 schematically shows a finished coating according to a first embodiment. The surface of the metal 1 has been treated with particle abrasion, and a layer of fabric 2 (the Figure showing the fibres in two directions) with resin has been applied closely to the surface 1. A fibre rowing 3 has then been applied on the fabric, and the space between the rowing filled with particles 4 of bioactive material. A second fibre fabric 5 impregnated with resin has then been applied to cover the rowing and particles, and the fabric 5 has a mesh size such that the openings 6 in the fabric are smaller than the average size of the particles 4.
Figures 2a and 2b schematically show a total hip prostheses partially coated according to a second embodiment. Figure 2a shows the hip prostheses 7 as a side view, and Figure 2b as a cross-sectional view. The rowing 3 has been applied at angles of approximately 45° with respect to the longitudinal direction of the prostheses 7 (which is also the main direction of load once the prosthesis is in place). In this embodiment, the distance between two rowings is 10 mm and the diameter of the rowing is 0.6 mm. The average size of the particles is 300-500 µm and the openings in the second fabric are less than 300 µm. In Figure 2b, it can be seen that the rowing is equally distributed around the prostheses.

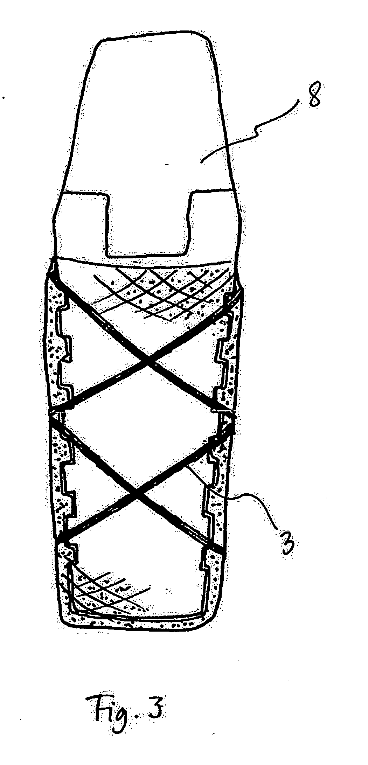

Figure 3 schematically shows a dental implant 8 partially coated according to a third embodiment. In this embodiment, the rowings are also placed at angles of about 45° with respect to the longitudinal direction of the dental implant, but the rowings are closer to each other, about 4 mm apart, and its thickness is about 0.3 mm, thus the particle size is also smaller, on average 200-300 µm and the openings in the second, outermost fabric are thus less than 200 µm in diameter.

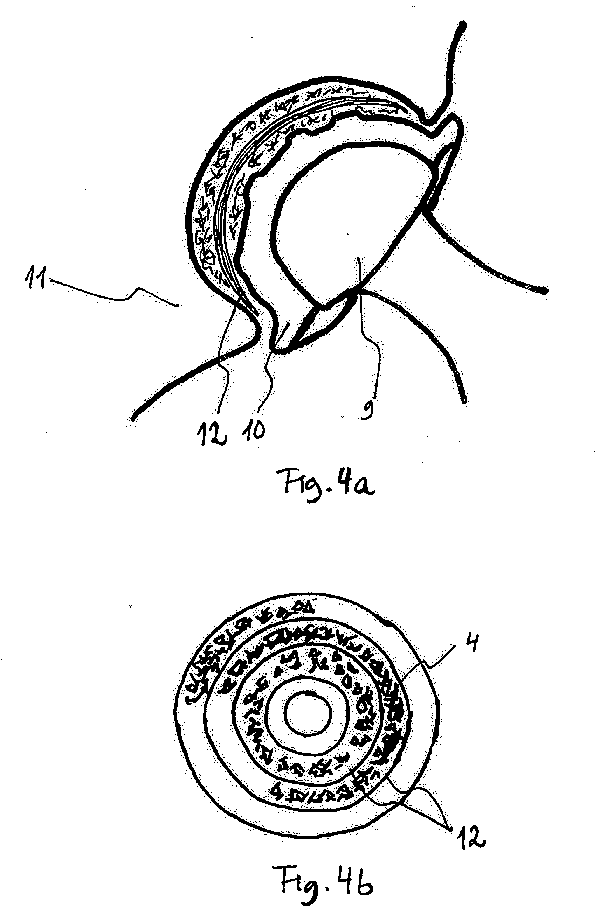

Figures 4a and 4b schematically show an acetabular cup system coated according to a fourth embodiment. The Figure 4a shows an acetabular cup system 10 and a femoral head 9, the acetabular cup system being used in connection with a total hip prosthesis and attached to the hip bone 11. In this embodiment, the bundles of unidirectional fibres 12 are arranged circularly on the surface of the cup, following the shape of the margins of the shell of the supporting acetabular cup system. The distance between each fibre bundle is 8 mm and space between the layers is 1.0 mm, corresponding to the diameter of the fibre bundles. This space of 1.0 mm allows a higher volume of new bone to grow into the space between the coating layers and thereby to provide a better stability for the implant in long term. Figure 4b shows the acetabular cup viewed from above, where it can be seen the arrangement of the fibre bundles 12 and that part of the remaining surface has already been filled with particles of bioactive material 4.

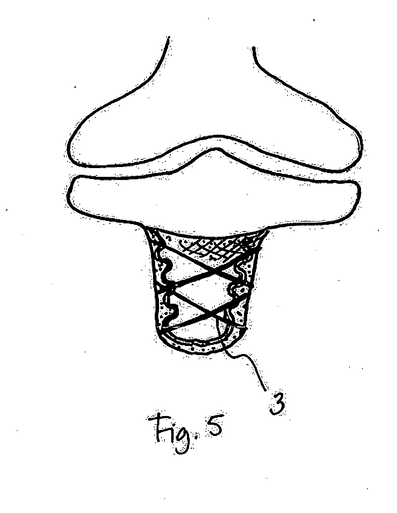

Figure 5 schematically shows a knee implant coated according to a fifth embodiment, where the fibre rowings 3 are also arranged at angles of about 45° with respect to the longitudinal direction of the implant. The distance of the rowings to each other is 5 mm and the space between the layers is 0.6 mm (i.e. the diameter of the fibre rowing).

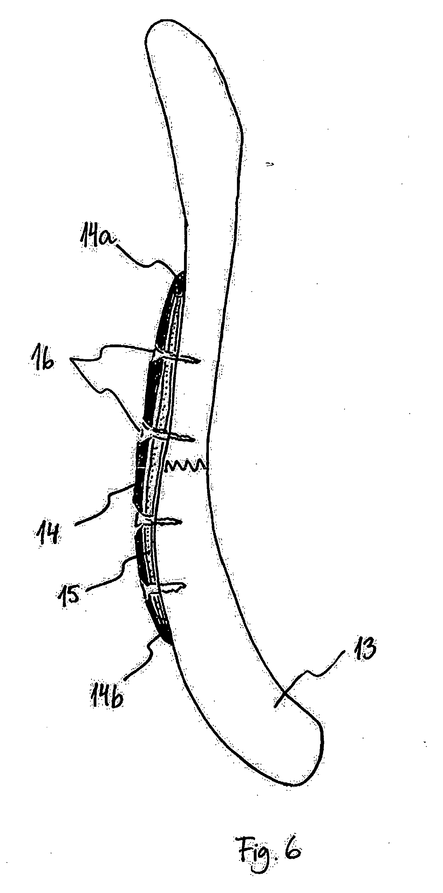

Figure 6 schematically shows fixation of a fractured clavicle bone 13 by a metal plate 14 in which the bone contacting surface has been coated according to the present invention. The metal plate 14 is in contact with the bone 13 at the ends 14a and 14b of the plate and the coating layers 15 are positioned at the concavity of the metal plate 14. The fixation screws 16 go through the metal and the fibre coating layer. The compression induced by the screws 16 is transmitted to the bone through the contact points at the ends of the plate.

### EXPERIMENTAL PART

Manufacture of a coating on a hip prostheses made of titanium

The titanium surface of a stem of a total hip prostheses was air-particle abraded with Rocatec (3M-Espe, Germany) silica coated alumina particles using an air-pressure of 280 kPa. The air-particle abrasion led to a roughened titanium surface containing silica.

A silane solution based on gamma methacryloxy propyl trimethoxy silane and ethanol with a silane content of 1 wt-% was used for silanization. The components were mixed, the pH of the solution was adjusted to 4 with 1 M acetic acid and the silane solution was allowed to hydrolyse for 1 h at room temperature. Thereafter the silane solution was applied to the surface of air-particle abraded titanium and allowed to polymerise, i.e. to polycondensate with silica on the surface at a temperature of 100 °C for one hour.

In the following step, a layer of resin impregnated S3-glass fibre fabric (220 g/m²) was applied on the surface, the resin being a photopolymerisable resin of bisphenol-A-glycidyl dimethacrylate and triethylene glycol dimethacrylate. The resin was then light polymerised in normal atmospheric conditions which lead to an oxygen inhibited layer on the treated surface, thus allowing the next composite layers to attach completely to this first layer. Next, resin impregnated unidirectional glass fibre rowings (same resin as above) were placed on the first laminate to form interconnective elements, which bind the first laminate layer (closest to titanium) and the outermost layer together, once the coating is finished. The thickness of the interconnective element (the rowing) was 0.6 mm, thus leading to a space of 0.6 mm between the inner and outer layers. After light polymerization of the interconnective element, which attached it to the first layer, particulates of bioactive glass S53P4 (average particle size 300-500 µm) were applied between the interconnective elements on the first layer and covered with a S3-glass fibre fabric having a mesh-like structure with openings (mesh size) of less than 300 µm, impregnated with the same resin as above. The resin in the fabric was then photopolymerised to cure the resin of the outermost layer and to adhere it to the interconnective elements. The particulates of bioactive glass were thus covered with a layer of mesh-like glass fabric. In a last step, the coated implant was placed under vacuum at a temperature of 120 °C, in order to post-cure the resins. Prior to the use of the coated implant, the implant was sterilised by a hydrogen peroxide system.

### Manufacture of a fixation plate for clavicle bone

A titanium alloy metal plate with screw holes was milled to have a concavity (1.4 mm in depth) on the bone contacting side of the plate. Marginal areas of the concavity formed undercuts for the fibre bundle layer for mechanical retaining. The concavity was further air-particle abraded with Rocatec (3M-Espe, Germany) silica coated alumina particles using an air-pressure of 280 kPa. The air-particle abrasion led to a roughened surface to be silanated.

A first layer S3-glass fibre fabric (thickness of 0.4 mm) was laminated by bis-GMA-TEGDMA (50:50) monomer system to the bottom of the concavity. The monomer system was light polymerised in order to have an initial attachment for the first fibre layer. One interconnective, bar shaped element of unidirectional S3-glass fibre roving is laminated to the first fibre fabric layer with orientation along the long axis of the metal plate. The thickness of the bar was 0.7 mm. Particulates of bioactive glass S53P4 were powdered to the rest of the space on the concavity and covered with a layer of S3-glass fibre fabric (thickness of 0.4 mm) with open holes (fibre mesh). This layer was pressed against the interconnective bar and the margins of the first fibre fabric layer and initially light polymerised. The plate with the coating on its inner surface was post-cured at the temperature of 120 °C for 30 minutes. Holes for screws were drilled through the material. The fixation plate was cleaned with compressed air blow and sterilised with H₂O₂ plasma.

## Claims

1. A method of coating a surface, comprising the steps of
- abrading the surface (1) to be coated with particles to form an abraded surface,
- forming and/or arranging a composite structure on the abraded surface, the composite structure comprising
- a first fibre fabric (2) impregnated with a first resin,
- at least one fibre bundle (3) impregnated with a second resin, the fibre bundle (3) being arranged according to a pattern forming in-between spaces, and in contact with the first fibre fabric (2),
- particles (4) of bioactive material arranged into said in-between spaces of the fibre bundle (3) and arranged in contact with the first fibre fabric,
- a second fibre fabric (5) impregnated with a third resin and arranged in contact with the fibre bundles (3) and at least part of the particles (4) of bioactive material, wherein the mesh size of the second fibre fabric (5) is smaller than the average diameter of the particles (4) of bioactive material,
- polymerising the resins of the composite structure.

2. Method according to claim 1, **characterised in that** the step of arranging the composite structure on the abraded surface (1) comprises the steps of
- applying the first resin to the abraded surface (1) to form a first resin coated surface,
- applying the first fibre fabric (2) onto the first resin coated surface,
- polymerising the first resin in atmospheric conditions to form an oxygen inhibited surface,
- applying the at least one fibre bundle (3) impregnated with the second resin according to the pattern to the oxygen inhibited surface,
- applying particles (4) of bioactive material to the oxygen inhibited surface into said in-between spaces of the fibre bundle (3),
- polymerising the second resin to form a semi-coated surface,
- applying the second fibre fabric (5) impregnated with the third resin to the semi-coated surface, and
- polymerising the third resin of the second fibre fabric (5) to form the coated surface.

3. A method according to claim 2, **characterised in that** the first fibre fabric (2) is impregnated with the first resin prior to applying it to the abraded surface.

4. Method according to claim 1, **characterised in that** the composite structure is formed separately and arranged on the abraded surface (11).

5. A method according to any of the preceding claims, **characterised in that** the step of abrading the surface (1) to be coated is carried out with particles containing silica and **in that** it further comprises, after abrading the surface (1) to be coated and prior the application of the composite structure, the steps of
- applying a solution containing a silane coupling agent to the abraded surface to form a solution coated surface, and
- polymerizing the solution to form a silanized surface.

6. A method according to any of the preceding claims, **characterised in that** the solution containing a silane coupling agent is allowed to hydrolyse before applying it to the abraded surface (1).

7. A method according to any of the preceding claims, **characterised in that** the second, third and fourth resins are photopolymerisable resins selected from the group consisting of dimethacrylate monomers.

8. A method according to any of the preceding claims, **characterised in that** the fibres used in the fabrics (2.5) and/or the fibre bundle (3) are glass fibres.

9. A method according to claim 8, **characterised in that** the glass fibres are made of a glass composition of S-glass, E-glass or bioactive glass.

10. A method according to any of the preceding claims, **characterised in that** the bioactive material is selected from the group consisting of bioactive glass, hydroxyapatite, tricalciumphosphate and mixtures thereof.

11. A method according to any of the preceding claims, **characterised in that** the diameter of the fibre bundle is 0.1-10 mm.

12. A method according to any of the preceding claims, **characterised in that** the average particle size of the bioactive material is 10-1000 µm.

13. A coating of a surface obtainable by the method of any of the claims 1-12.

14. An implant obtainable by at least partially coating its surface according to the method of any of the claims 1-12.

15. An implant according to claim 14, **characterised in that** the implant is selected from the group consisting of hip prostheses, acetabular cups, knee prostheses, intramedullary nails, fracture fixation plates and dental implants.

## Patentansprüche

1. Verfahren zur Beschichtung einer Oberfläche, das folgende Schritte umfasst:
- Schleifen der zu beschichtenden Oberfläche (1) mit Partikeln zum Bilden einer geschliffenen Oberfläche,
- Bilden und/oder Anordnen einer Verbundstruktur auf der geschliffenen Oberfläche, wobei die Verbundstruktur Folgendes umfasst:
- ein erstes Fasergewebe (2), das mit einem ersten Harz getränkt ist,
- mindestens ein Faserbündel (3), das mit einem zweiten Harz getränkt ist, wobei das Faserbündel (3) entsprechend einem Muster angeordnet ist, das Zwischenräume bildet, und das erste Fasergewebe (2) berührt,
- Partikel (4) aus bioaktivem Material, die in den Zwischenräumen des Faserbündels (3) angeordnet und das erste Fasergewebe berührend angeordnet sind,
- ein zweites Fasergewebe (5), das mit einem dritten Harz getränkt ist und die Faserbündel (3) und zumindest einen Teil der Partikel (4) aus bioaktivem Material berührend angeordnet ist, wobei die Maschenweite des zweiten Fasergewebes (5) kleiner ist als der mittlere Durchmesser der Partikel (4) aus bioaktivem Material,
- Polymerisieren der Harze der Verbundstruktur.

2. Verfahren nach Anspruch 1, **dadurch gekennzeichnet, dass** der Schritt des Anordnens der Verbundstruktur auf der geschliffenen Oberfläche (1) folgende Schritte umfasst:
- Aufbringen des ersten Harzes auf die geschliffene Oberfläche (1) zum Bilden einer ersten harzbeschichteten Oberfläche,
- Aufbringen des ersten Fasergewebes (2) auf die erste harzbeschichtete Oberfläche,
- Polymerisieren des ersten Harzes unter atmosphärischen Bedingungen zum Bilden einer sauerstoffinhibierten Oberfläche,
- Aufbringen des mindestens einen Faserbündels (3), das mit dem zweiten Harz getränkt ist, entsprechend dem Muster auf die sauerstoffinhibierte Oberfläche,
- Aufbringen von Partikeln (4) aus bioaktivem Material auf die sauerstoffinhibierte Oberfläche in die Zwischenräume des Faserbündels (3),
- Polymerisieren des zweiten Harzes zum Bilden einer halbbeschichteten Oberfläche,
- Aufbringen des zweiten Fasergewebes (5), das mit dem dritten Harz getränkt ist, auf die halbbeschichtete Oberfläche, und
- Polymerisieren des dritten Harzes des zweiten Fasergewebes (5) zum Bilden der beschichteten Oberfläche.

3. Verfahren nach Anspruch 2, **dadurch gekennzeichnet, dass** das erste Fasergewebe (2) mit dem ersten Harz getränkt wird, bevor es auf die geschliffene Oberfläche aufgebracht wird.

4. Verfahren nach Anspruch 1, **dadurch gekennzeichnet, dass** die Verbundstruktur separat geformt und auf der geschliffenen Oberfläche (1) angeordnet wird.

5. Verfahren nach einem der vorhergehenden Ansprüche, **dadurch gekennzeichnet, dass** der Schritt des Schleifens der zu beschichtenden Oberfläche (1) mit Partikeln durchgeführt wird, die Siliziumdioxid enthalten, und dass es ferner nach dem Schleifen der zu beschichtenden Oberfläche (1) und vor dem Aufbringen der Verbundstruktur folgende Schritte umfasst:
- Auftragen einer Lösung, die einen Silan-Haftvermittler enthält, auf die geschliffene Oberfläche zum Bilden einer lösungsbeschichteten Oberfläche, und
- Polymerisieren der Lösung zum Bilden einer silanisierten Oberfläche.

6. Verfahren nach einem der vorhergehenden Ansprüche, **dadurch gekennzeichnet, dass** die Lösung, die einen Silan-Haftvermittler enthält, hydrolysiert wird, bevor sie auf die geschliffene Oberfläche (1) aufgetragen wird.

7. Verfahren nach einem der vorhergehenden Ansprüche, **dadurch gekennzeichnet, dass** das zweite, dritte und vierte Harz mittels Photopolymerisation polymerisierbare Harze sind, die aus der Gruppe bestehend aus Dimethacrylatmonomeren ausgewählt sind.

8. Verfahren nach einem der vorhergehenden Ansprüche, **dadurch gekennzeichnet, dass** die in den Geweben (2, 5) und/oder dem Faserbündel (3) verwendeten Fasern Glasfasern sind.

9. Verfahren nach Anspruch 8, **dadurch gekennzeichnet, dass** die Glasfasern aus einer Glaszusammensetzung aus S-Glas, E-Glas oder bioaktivem Glas gefertigt sind.

10. Verfahren nach einem der vorhergehenden Ansprüche, **dadurch gekennzeichnet, dass** das bioaktive Material aus der Gruppe bestehend aus bioaktivem Glas, Hydroxyapatit, Tricalciumphosphat und Mischungen daraus ausgewählt ist.

11. Verfahren nach einem der vorhergehenden Ansprüche, **dadurch gekennzeichnet, dass** der Durchmesser des Faserbündels 0,1 bis 10 mm beträgt.

12. Verfahren nach einem der vorhergehenden Ansprüche, **dadurch gekennzeichnet, dass** die mittlere Korngröße des bioaktiven Materials 10 bis 1000 µm beträgt.

13. Beschichtung einer Oberfläche, die sich mit dem Verfahren nach einem der Ansprüche 1 bis 12 erhalten lässt.

14. Implantat, das sich erhalten lässt, indem seine Oberfläche zumindest teilweise nach dem Verfahren nach einem der Ansprüche 1 bis 12 beschichtet wird.

15. Implantat nach Anspruch 14, **dadurch gekennzeichnet, dass** das Implantat aus der Gruppe bestehend aus Hüftprothesen, Hüftgelenkpfannen, Knieprothesen, intramedullären Nägeln, Platten zur Bruchfixierung und Zahnimplantaten ausgewählt ist.

## Revendications

1. Un procédé de revêtement d'une surface, comprenant les étapes consistant à
- abraser la surface (1) à revêtir avec des particules afin de former une surface abrasée,
- former et/ou disposer une structure composite sur la surface abrasée, la structure composite comprenant
- un premier tissu de fibres (2) imprégné d'une première résine,
- au moins un faisceau de fibres (3) imprégné d'une deuxième résine, le faisceau de fibres (3) étant agencé selon un motif formant des espaces intermédiaires et en contact avec le premier tissu de fibres (2),
- des particules (4) de matériau bioactif disposées dans lesdits espaces intermédiaires du faisceau de fibres (3) et disposées en contact avec le premier tissu de fibres,
- un second tissu de fibres (5) imprégné d'une troisième résine et disposé en contact avec les faisceaux de fibres (3) et au moins une partie des particules (4) de matériau bioactif, dans lequel l'ouverture de maille du second tissu de fibres (5) est inférieure au diamètre moyen des particules (4) de matériau bioactif,
- polymériser les résines de la structure composite.

2. Procédé selon la revendication 1, **caractérisé en ce que** l'étape consistant à disposer la structure composite sur la surface abrasée (1) comprend les étapes consistant à
- appliquer la première résine sur la surface abrasée (1) pour former une première surface revêtue de résine,
- appliquer le premier tissu de fibres (2) sur la première surface revêtue de résine,
- polymériser la première résine dans des conditions atmosphériques pour former une surface inhibée par l'oxygène,
- appliquer l'au moins un faisceau de fibres (3) imprégné de la deuxième résine sur la surface inhibée par l'oxygène selon le motif,
- appliquer des particules (4) de matériau bioactif sur la surface inhibée par l'oxygène dans lesdits espaces intermédiaires du faisceau de fibres (3),
- polymériser la deuxième résine pour former une surface semi-revêtue,
- appliquer le second tissu de fibres (5) imprégné de la troisième résine sur la surface semi-revêtue, et
- polymériser la troisième résine du second tissu de fibres (5) pour former la surface revêtue.

3. Procédé selon la revendication 2, **caractérisé en ce que** le premier tissu de fibres (2) est imprégné de la première résine avant de l'appliquer sur la surface abrasée.

4. Procédé selon la revendication 1, **caractérisé en ce que** la structure composite est formée séparément et disposée sur la surface abrasée (1).

5. Procédé selon l'une quelconque des revendications précédentes, **caractérisé en ce que** l'étape consistant à abraser la surface (1) à revêtir est exécutée avec des particules contenant de la silice et **en ce qu'**il comprend en outre, après l'abrasion de la surface (1) à revêtir et avant l'application de la structure composite, les étapes consistant à
- appliquer une solution contenant un agent adhésif au silane sur la surface abrasée pour former une surface revêtue de solution, et
- polymériser la solution pour former une surface silanisée.

6. Procédé selon l'une quelconque des revendications précédentes, **caractérisé en ce que** la solution contenant un agent adhésif au silane est permis de s'hydrolyser avant d'être appliquée sur la surface abrasée (1).

7. Procédé selon l'une quelconque des revendications précédentes, **caractérisé en ce que** les deuxième, troisième et quatrième résines sont des résines photopolymérisables choisies dans le groupe constitué des monomères de diméthacrylate.

8. Procédé selon l'une quelconque des revendications précédentes, **caractérisé en ce que** les fibres utilisées dans les tissus (2, 5) et/ou le faisceau de fibres (3) sont des fibres de verre.

9. Procédé selon la revendication 8, **caractérisé en ce que** les fibres de verre sont fabriquées en une composition de verre constituée de verre S, de verre E ou de verre bioactif.

10. Procédé selon l'une quelconque des revendications précédentes, **caractérisé en ce que** le matériau bioactif est choisi dans le groupe constitué du verre bioactif, de l'hydroxyapatite, du phosphate tricalcique et de mélanges de ceux-ci.

11. Procédé selon l'une quelconque des revendications précédentes, **caractérisé en ce que** le diamètre du faisceau de fibres est de 0,1 à 10 mm.

12. Procédé selon l'une quelconque des revendications précédentes, **caractérisé en ce que** la taille de particule moyenne du matériau bioactif est de 10 à 1000 µm.

13. Un revêtement d'une surface pouvant être obtenu par le procédé selon l'une quelconque des revendications 1 à 12.

14. Un implant pouvant être obtenu en revêtant au moins partiellement sa surface selon le procédé selon l'une quelconque des revendications 1 à 12.

15. Implant selon la revendication 14, **caractérisé en ce que** l'implant est choisi dans le groupe constitué des prothèses de la hanche, des cotyles, des prothèses du genou, des clous centromédullaires, des plaques de fixation de fracture et des implants dentaires.
